# FASCICULE DE BREVET EUROPEEN

(11) **EP 0 878 175 B1**
(45) Date de publication et mention de la délivrance du brevet: **10.12.2003**
(21) Numéro de dépôt: 98401087.6
(22) Date de dépôt: 05.05.1998
(51) Int. Cl.: A61F 2/06

(54) **Système de réparation d'un conduit anatomique par un implant a ouverture progressive**
System zum Ausbessern eines Körpergefässes durch ein Implantat mit einer sich stufenweise ausweiterbaren Öffnung
System for repairing a body vessel using a progressively opening implant

(30) Priorité: 12.05.1997 FR 9705783
(43) Date de publication de la demande: 18.11.1998
(73) Titulaire: B. Braun Celsa, 92100 Boulogne-Billancourt (FR)
(72) Inventeur: Nadal, Guy M., 86000 Poitiers (FR)
(74) Mandataire: Lerner, François

(56) Documents cités:
- WO-A-95/01761
- WO-A-96/18361
- US-A- 5 405 378

## Description

L'invention concerne un sytème ou dispositif de réparation d'un conduit anatomique dans lequel circule un fluide.

Il s'agit en particulier de réparer un vaisseau sanguin.

Le vaisseau "à réparer" peut typiquement être affecté d'un ou plusieurs anévrisme(s) ou d'une dégénérescence nécessitant la pose d'une tubulure destinée à canaliser le sang dans la(les) portion(s) endommagée(s), évitant ainsi de laisser la pression sanguine s'appliquer contre les parois fragilisées du vaisseau.

On a jusqu'à présent proposé de nombreux implants vasculaires de traitement d'anévrismes.

Depuis quelques années, on favorise la pose de ces implants par voie endoluminale percutanée.

Parmi ces systèmes existants, WO-A-96/18361 propose en particulier un système comprenant :
- un implant ayant un corps propre à canaliser le fluide considéré suivant un tube unique ou ramifié s'étendant globalement le long d'un axe principal de l'implant, au moins dans une configuration de ce dernier, lequel présente une première configuration pour être contenue à l'intérieur d'une gaine flexible, à fins d'implantation dans le conduit, ou une seconde configuration, une fois l'implant disposé dans le conduit hors de la gaine, la première configuration présentant un encombrement radial par rapport audit axe principal inférieur à la seconde configuration et ledit corps ayant une longueur suivant cet axe ;
- un dispositif de pose de l'implant dans le conduit, le dispositif comprenant :
   ∗ ladite gaine flexible,
   ∗ au moins un lien de maintien, pour maintenir le corps sensiblement dans la première configuration de l'implant,
   ∗ et des moyens de libération du lien, pour permettre à l'implant de passer de sa première à sa seconde configurations.

Plus précisément encore, WO-A-96/18361 prévoit l'utilisation combinée de liens de maintien du corps de l'implant dans l'état "radialement resserré" de cet implant et de moyens de libération desdits liens permettant de contrôler tant l'introduction du corps dans la gaine de pose, que l'étape d'implantation proprement dite de l'implant dans le conduit anatomique récepteur.

Mais en réalité, l'action des liens s'opère sur deux stents (ou élargisseurs) tubulaires radialement déployables, disposés respectivement en parties "supérieure" (distale) et "inférieure" (proximale) de l'implant.

Or, entre ces deux niveaux "supérieur" et "inférieur", le fil de libération utilisé pour permettre aux stents de s'expanser radialement passe à l'intérieur d'un cathéter central autour duquel l'implant a été disposé, à l'intérieur de la gaine de pose.

En outre, d'autres fils ou filaments sont associés aux moyens de libération et de maintien déjà évoqués, ce qui rend la solution de WO-A-96/18361 complexe à réaliser et délicate à utiliser.

L'invention a pour objet de proposer une solution plus simple, permettant en particulier de contrôler les conditions de pose au regard du moment où le corps tubulaire de l'implant va s'ouvrir dans son conduit récepteur, tout en limitant les risques que l'on puisse mal positionner le corps dans son implant de pose.

En d'autres termes, l'invention se propose d'apporter une solution aux conditions rigoureuses tant de mise en place de l'implant (et en particulier de son corps "tubulaire") dans la gaine de pose, que d'ouverture radiale lorsque le corps de l'implant doit donc venir au moins localement se plaquer contre la paroi de son conduit récepteur.

Pour cela, la solution de l'invention consiste en ce que le système de réparation ci-avant énoncé soit tel que le(s) lien(s) de maintien du corps de l'implant s'étend(ent) autour d'au moins une portion majeure de la longueur du corps, pour le maintenir alors sensiblement dans ladite première configuration de l'implant, sur cette portion majeure.

Ainsi, si on n'exclut bien entendu pas dans l'invention d'utiliser un ou plusieurs liens de serrage pour maintenir dans la première configuration (radialement "resserrée") les moyens qui peuvent être utilisés sur l'implant pour plaquer le corps tubulaire contre la paroi interne du conduit récepteur (stents), on optimise là en particulier tant la mise en place que les conditions d'ouverture radiale du corps de cet implant.

A cet égard, une caractéristique complémentaire prévoit que le(s) lien(s) en question enlacera(ront) ce corps, dans la gaine, en formant, entre eux ou en coopération avec les moyens de libération, une série d'entrelacs libérables, sur au moins ladite "portion majeure" du corps.

Ainsi, les liens en question entoureront plusieurs fois le corps en le serrant, de préférence sur un cathéter intérieur autour duquel l'ensemble de l'implant aura préalablement été disposé.

Pour en particulier contrôler la bonne ouverture de ce corps, une caractéristique complémentaire de l'invention prévoit que les moyens de libération et les entrelacs coopéreront de préférence ensemble pour que ces entrelacs soient libérés progressivement par les moyens de libération, les uns après les autres.

Ceci est particulièrement intéressant à l'intérieur de vaisseaux tortueux ou lors de la pose d'un implant bifurqué, permettant ainsi au praticien d'optimiser d'abord le positionnement de l'implant dans son conduit récepteur, avant de l'ouvrir, dans un second temps.

C'est d'ailleurs dans cette optique, qu'une caractéristique complémentaire de l'invention prévoit qu'avantageusement les moyens de libération seront liés au(x) lien(s) à une extrémité de la ligature réalisée, de manière que sa libération s'opère, depuis cette extrémité, uniquement dans un sens.

En effet, c'est alors sensiblement à partir d'une extrémité du corps que l'on pourra déclencher l'ouverture de celui-ci (en particulier depuis son extrémité distale, en direction de son extrémité proximale), ce qui d'ailleurs simplifiera les conditions pratiques de réalisation de la liaison "lien(s)/moyens de libération."

Concernant la manière de réaliser le maintien du corps par le(s) lien(s), une première solution propose que ce(s) lien(s) définit(ssent) une succession de boucles se tenant essentiellement les unes les autres, en s'échelonnant au moins sur ladite portion majeure de la longueur du corps, pour former une chaîne.

Une seconde solution consiste à prévoir l'utilisation en tant que moyen de libération, d'au moins un élément allongé, flexible mais ayant une rigidité supérieure à celle des liens et s'étendant au moins sur ladite portion majeure de la longueur du corps tubulaire, le(s) lien(s) passant sensiblement "en épingle recourbée" autour de l'élément allongé, périodiquement d'un côté et de l'autre de celui-ci, en s'échelonnant au moins sur ladite portion majeure de la longueur du corps, jusqu'à former un noeud libérable dans l'environnement d'au moins une extrémité de liaison entre l'élément allongé en cause et le lien considéré, permettant de libérer le(chaque) lien par une action sur le noeud et lesdits moyens de libération qui assurent un échappement du premier vis-à-vis des seconds.

Si l'on retient cette solution, une autre caractéristique de l'invention conseille d'ailleurs que ledit noeud libérable soit formé vers l'extrémité distale de l'élément allongé, lequel s'étendra lui-même jusque vers l'extrémité distale du corps (et donc de l'implant), de telle sorte que le noeud en question soit propre à s'échapper de l'élément au moment où l'on viendra tirer celui-ci vers l'arrière, depuis l'extérieur du corps du patient (extrémité proximale des moyens de pose), libérant ainsi progressivement les "épingles doubles" successives à mesure qu'on retirera ledit élément.

Une description plus détaillée de l'invention va maintenant être fournie en relation avec les dessins annexés, dans lesquels :
les figures 1 et 2 présentent schématiquement un premier mode de réalisation d'un implant vasculaire, respectivement en configurations radialement déployée et radialement resserrée,
les figures 3, 4 et 5 montrent schématiquement trois alternatives de réalisation d'un tel implant, à chaque fois en position radialement déployée,
les figures 6, 7, 8, 9 et 13 montrent essentiellement un dispositif de pose utilisable pour introduire l'un des implants précédents dans son conduit d'implantation, les figures 6, 7 et 13 montrant le dispositif en situation.
la figure 9 est une vue locale agrandie de la figure 8, à l'endroit repéré IX sur cette figure 8,
la figure 10 est une vue encore agrandie de la partie située en haut de la figure 9,
les figures 11 et 12 montrent (en vue locale agrandie pour la figure 12) le principe de laçage ou nouage retenu pour le corps de l'implant sur les figures 9 et 10,
la figure 14 montre l'implant en situation dans son conduit récepteur,
et les figures 15 et 16 montrent (en vue locale agrandie pour la figure 16) une alternative de nouage pour enlacer ledit corps.

Par souci de clarté, on ne traitera ci-après que du cas particulier d'un implant vasculaire destiné au traitement d'anévrismes, même si d'autres affections et conduits anatomiques pourraient être concernés par l'invention.

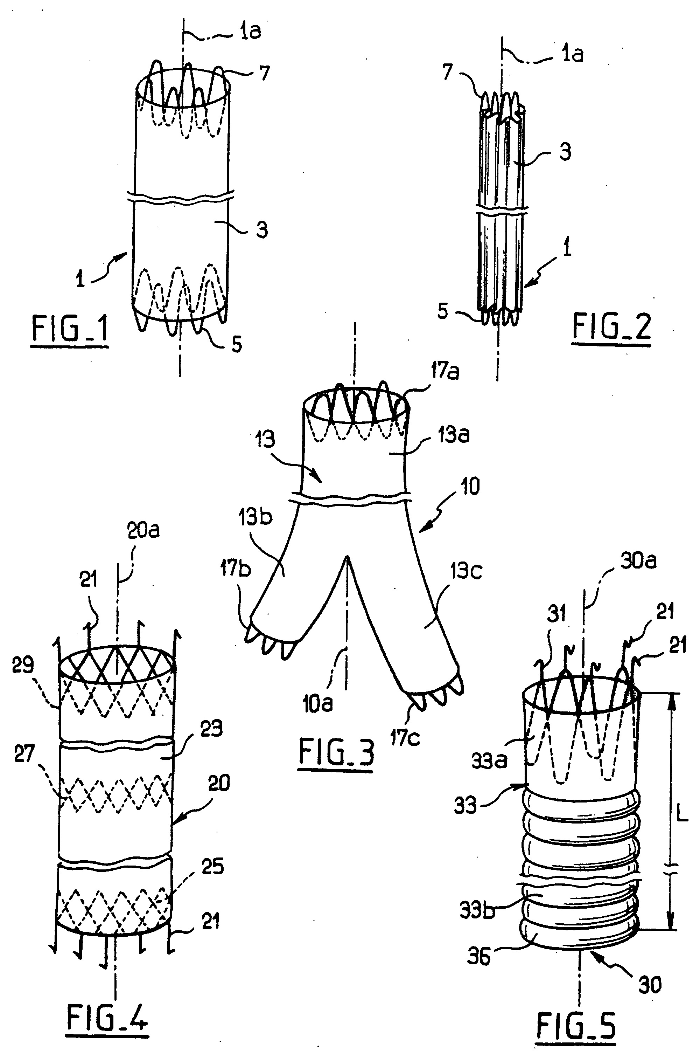

On notera également que l'objet des figures 1 à 6 est essentiellement de figurer quelques exemples de réalisation d'un tel implant, étant précisé que pratiquement toutes les configurations existantes à ce sujet pourraient être utilisées, qu'il s'agisse d'un implant à armature (stent) auto-expansible (c'est-à-dire ayant naturellement la capacité de se déformer radialement de façon élastique depuis un premier diamètre resserré jusqu'à un second diamètre élargi) ou d'un implant à armature radialement déployable sous l'effet d'une force interne pouvant être créée par l'intermédiaire d'un ballon gonflable que l'on vient, le moment venu, gonfler à l'intérieur de l'implant (comme dans US-A-4 733 665, par exemple).

Typiquement, le manchon ou corps 3 est un tissé ou un non tissé sans tenue mécanique propre. Dans sa forme radialement déployée de la figure 1, il se présente comme un tube cylindrique de section circulaire et d'axe 1a confondu avec celui de l'implant 1. Il présente une paroi continue.

Eventuellement, une portion intermédiaire du manchon 3 pourrait être gaufrée pour résister à la pliure ou au vrillage.

Axialement, le manchon 3 peut présenter une longueur comprise entre environ 6 cm et 16 cm, avec un diamètre dans son état "radialement déployé" de la figure 1 compris entre environ 5 mm (implantation iliaque) et 45 mm (implantation aortique). Sa structure de paroi peut être réalisée en polyéthylène téréphtalate (PET), ou plus généralement en polyester ou encore en PTFE, avec une épaisseur de paroi de l'ordre 0,10 mm à 0,80 mm.

Pour son implantation par une gaine de petit diamètre (en particulier par voie endoluminale percutanée) et pour assurer le placage de l'implant contre la paroi du conduit récepteur, deux moyens-ressorts tubulaires 5, 7, ont été prévus, respectivement disposés vers les extrémités proximale et distale du manchon 3. Il s'agit en l'espèce de deux stents conformés en zigzags définissant chacun un anneau fermé sur lui-même, à la manière de ce qui est prévu dans EP-A-177 330 ou dans WO-A-96/18361. Pour réaliser ces stents, on peut utiliser un fil métallique (par exemple en acier inoxydable) pouvant avoir un diamètre de l'ordre de 0,5 mm, en vue de réaliser un anneau naturellement expansible radialement pour évoluer entre un diamètre d'anneau restreint (figure 2) d'environ 2 mm à 9 mm et le diamètre naturellement élargi déjà indiqué (figure 1).

Dans la position radialement contrainte de la figure 2, on notera que le manchon 3 présente des plis longitudinaux.

Sur la figure 3, on retrouve un implant 10 du même type que celui des figures 1 et 2, à la principale différence près que l'implant 10 est bifurqué. Pour cela, le manchon repéré ici 13 présente un tronçon principal 13a qui se ramifie en deux jambes 13b, 13c, l'une pouvant être plus courte que l'autre, donnant au tube 13 déployé une forme générale de "Y" inversé.

A l'extrémité libre de chacun des tronçons 13a, 13b, 13c, un "élargisseur" respectivement 17a, 17b, 17c, conformé comme les stents 5, 7, a été au moins partiellement glissé à l'intérieur du tronçon correspondant auquel il peut être lié par tout moyen, tel que des liens de suture ou des agrafes.

Pour poser l'implant 10 dans son conduit récepteur, via un dispositif d'implantation par voie endoluminale, on resserre radialement chacun des élargisseurs et on plaque les deux jambes 13b, 13c, l'une contre l'autre de manière à disposer le tout sensiblement suivant l'axe général 10a.

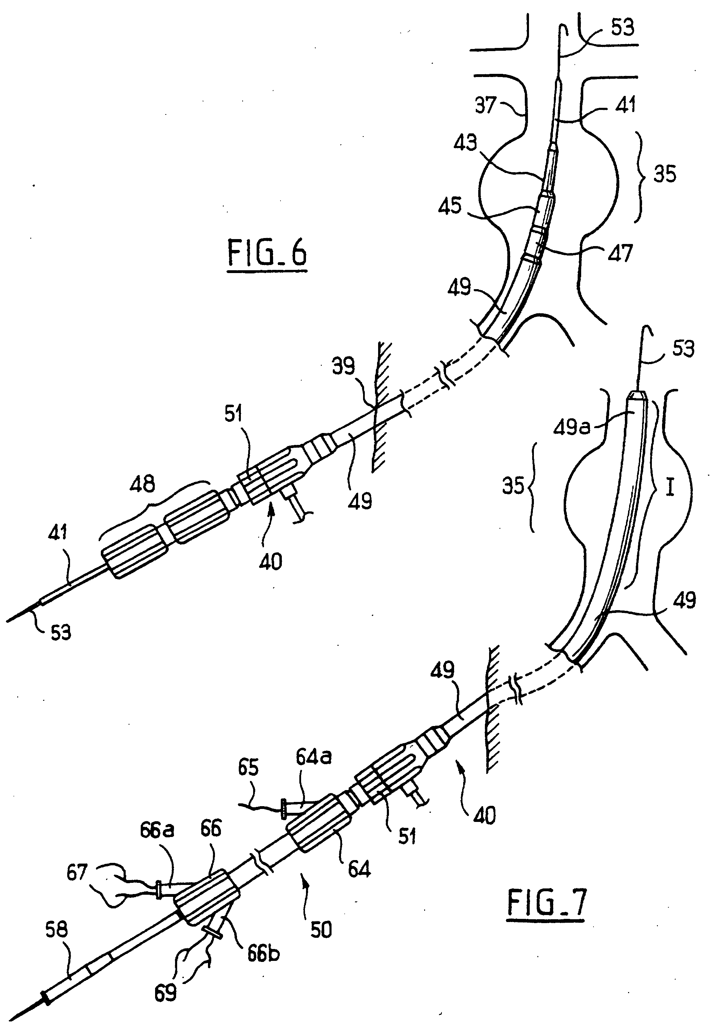

Sur la figure 4, l'implant 20 se présente à nouveau comme un tube unique d'axe longitudinal 20a. Mais en l'espèce, l'armature de déploiement radial du manchon 23 (du même type que le manchon 3) est constituée par trois tubes 25, 27, 29, formés chacun d'une pluralité d'éléments allongés s'intersectant constitués d'une pluralité de fines barres fixées les unes aux autres à leurs points de croisement, de manière à former une sorte de grillage radialement déformable sous l'effet d'une force interne (ballon ou équivalent voir US-A-4 733 665).

Sur la figure 4, on a par ailleurs repéré en 21 des crochets d'attache fixés respectivement aux tronçons proximal 25 et distal 29 de l'armature pour assurer l'accrochage de l'implant 20 dans la paroi de son conduit récepteur, en position radialement déployée. Bien entendu, de tels crochets d'attache peuvent être prévus sur les variantes des figures 1, 3 ou 5.

Sur la figure 5 justement, est représentée une autre variante d'implant pour anévrisme, 30. L'illustration schématise un implant tel que divulgué dans PCT/FR97/00734.

On remarque ainsi que l'implant vasculaire 30 comprend une armature tubulaire proximale 31 présentant un ou plusieurs niveaux d'enroulement filamentaire en zigzags. Cette armature métallique est auto-expansible, de sorte qu'en l'absence de contrainte radiale, elle fait se déployer radialement la portion distale 33a du manchon 33 qui s'étend autour d'elle. Cette première portion 33a est, à l'image du manchon 3, dépourvue de tenue mécanique propre, à la différence de la seconde portion 33b. Cette portion 33b, qui prolonge axialement la première, présente en effet naturellement une forme tubulaire radialement déployée, à la manière des substituts de vaisseau chirurgicaux (voir par exemple WO-A-88/06026 ou US-A-3 986 828).

La portion 33b est de préférence étanche au sang et est prévue pour être anastomosée au vaisseau récepteur considéré, vers son extrémité libre proximale 36.

Aucun stent (ou moyen de déploiement radial équivalent) n'existe en regard de l'essentiel au moins de cette portion anastomosable 33b qui peut être gaufrée et être fabriquée, par exemple, en Dacron (marque déposée).

En relation avec les figures 6 et suivantes, on va s'intéresser maintenant à la pose d'un des implants ci-avant présentés, par exemple celui de la figure 3, en vue du traitement d'un anévrisme aortique 35.

Le praticien peut réaliser la pose par une voie d'abord percutanée ou par dénudation vasculaire.

Supposons qu'une pose dans l'aorte 37 par voie percutanée, par exemple avec approche fémorale par l'artère iliaque droite a été retenue.

Après avoir incisé à travers la peau en 39, le praticien peut utiliser d'abord un premier ensemble de pose 40 tel qu'illustré sur la figure 6, comprenant un dilatateur flexible 41 autour duquel sont successivement disposées une série de gaines flexibles 43, 45, 47, 49 de diamètres croissants, à extrémité distale émoussée.

La gaine extérieure 49 est pourvue d'un embout proximal à valve interne, 51.

Les gaines intérieures 43, 45, 47, sont solidaires de la connexion proximale 48.

Par l'incision et à travers l'iliaque droite, jusque dans l'aorte, est d'abord glissé un guide-fil traditionnel 53. Le long du guide-fil est ensuite glissé le dilatateur 41 autour duquel sont alors introduites les gaines 43, 45, 47 et 49..

Une fois la gaine 49 correctement positionnée, avec son extrémité distale 49a légèrememt au-delà de l'anévrisme 35, le dilatateur 41 et les gaines intermédiaires 43, 45, 47 sont retirés, ainsi que le guide-fil 53.

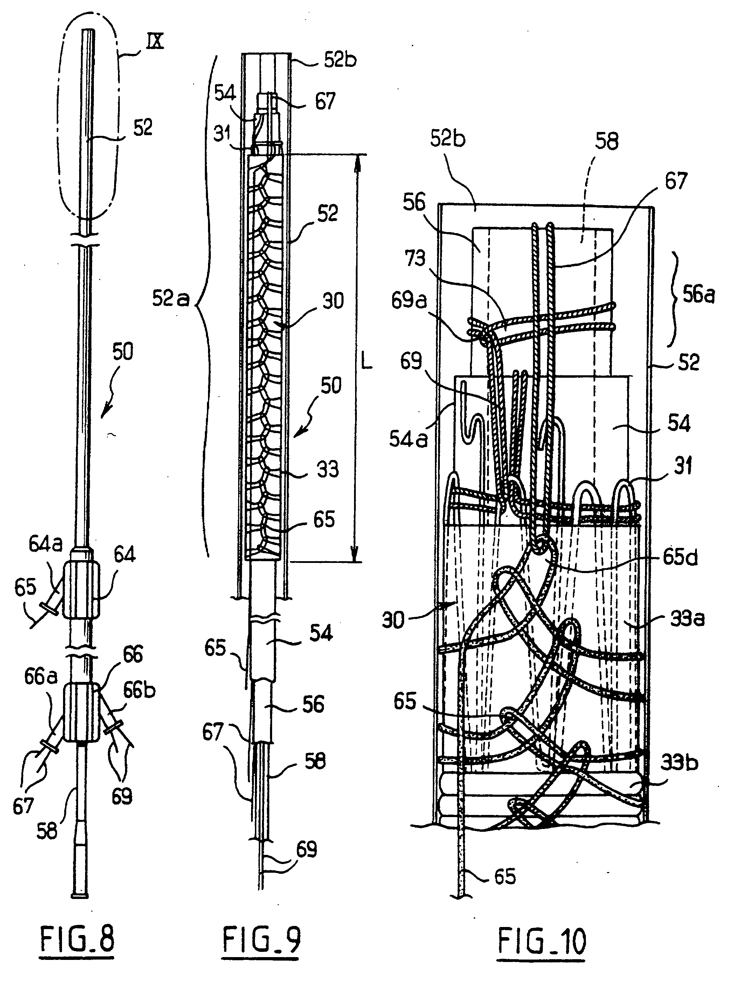

On vient alors glisser à l'intérieur de la gaine 49 un second ensemble de pose 50, comme montré sur la figure 7 et que l'on distingue plus clairement sur les figures 8 et 9.

L'ensemble 50 comprend une gaine extérieure 52 propre à être glissée à l'intérieur de la gaine 49 et qui renferme, sur l'essentiel au moins de sa longueur, trois tubes ou cathéters concentriques repérés successivement (du plus gros au plus petit) 54, 56 et 58 sur la figure 9, la figure 8 montrant que le cathéter central 58 est plus long que la gaine 52 et les autres cathéters intermédiaires (54 et 56), respectivement pourvus d'un embout proximal vissable 64, 66. A noter que l'embout 64 présente une sortie latérale 64a par où l'on voit déboucher un fil souple 65, tandis que l'embout 66 présente deux sorties latérales 66a et 66b par où débouchent les deux extrémités opposées de deux autres fils souples, respectivement 67 et 69 dont on va comprendre l'utilité. Les fils peuvent être en polyester ou en Nylon (marque déposée), tels des fils de suture.

A l'intérieur de la gaine 52, et plus particulièrement dans sa zone distale 52a proche de son extrémité distale libre 52b, a été préchargé l'implant 30 (voir figures 9 et 10).

Plus précisément, l'implant a été mis en place entre la gaine 52 et le cathéter 54, avec sa partie distale de manchon 33a soutenue par l'armature 31 prête à sortir la première.

Pour maintenir l'implant 30, et en particulier son corps formant manchon 33, à l'intérieur de la gaine 52 qui présente typiquement un diamètre interne D de l'ordre de 2 mm à 9 mm), le fil 65 dont on a déjà parlé a été passé à la manière d'un lien de serrage autour de l'essentiel au moins de la longueur axiale L du corps 33.

Ce lien (mais il pourrait y en avoir plusieurs) a pour première fonction de figer, à l'intérieur de la gaine 52, la position du manchon 33 qui est ainsi placé de façon optimale dans son état "radialement resserré", évitant ainsi les mauvaises pliures et autres états inappropriés.

Ainsi, le lien 65 s'étend-il au moins autour de la portion de gaine non soutenue par l'armature 31, d'autant plus qu'en l'espèce, la portion en question (33b) a naturellement tendance à s'ouvrir radialement et qu'il faut donc la contraindre pour que, correctement pliée, elle occupe un encombrement radial suffisamment réduit pour pouvoir être introduite à l'intérieur de la gaine 52.

Sur les figures 9, 10, 11 et 15, le lien 65 s'étend toutefois aussi autour de la zone distale 33a, c'est-à-dire par dessus le stent 31, sur sensiblement toute la longueur axiale de l'implant 30.

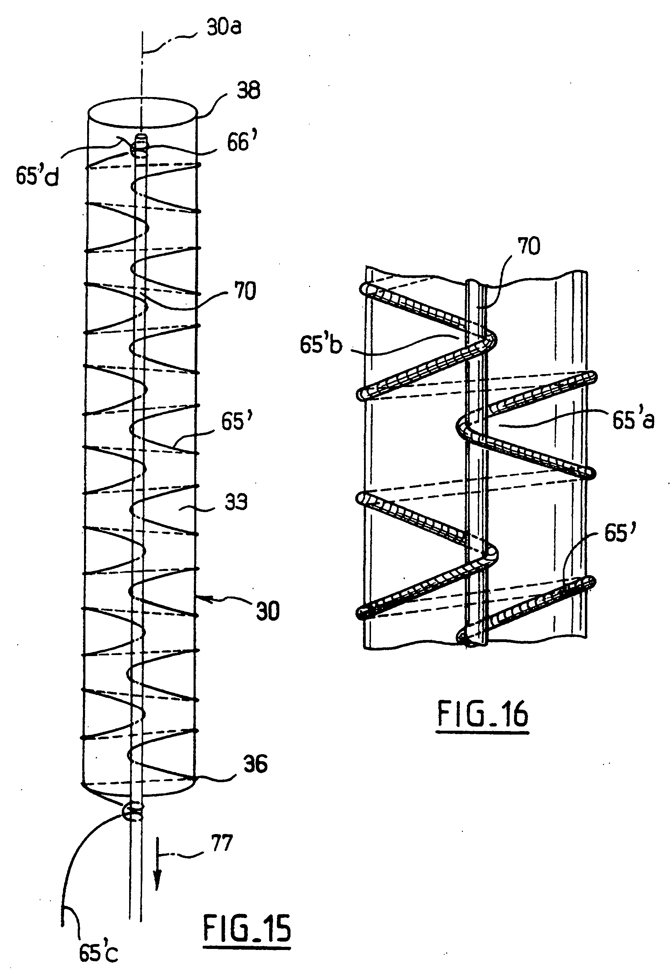

Pour assurer le serrage radial autour du manchon, le lien l'enlace en formant une série d'entrelacs réguliers le long de ce corps, ces entrelacs étant libérables, en l'espèce par un moyen annexe de libération qui peut se présenter sous différentes formes, telles que le fil souple 67 des figures 9 à 12 ou la tige plus rigide 70 des figures 15 et 16.

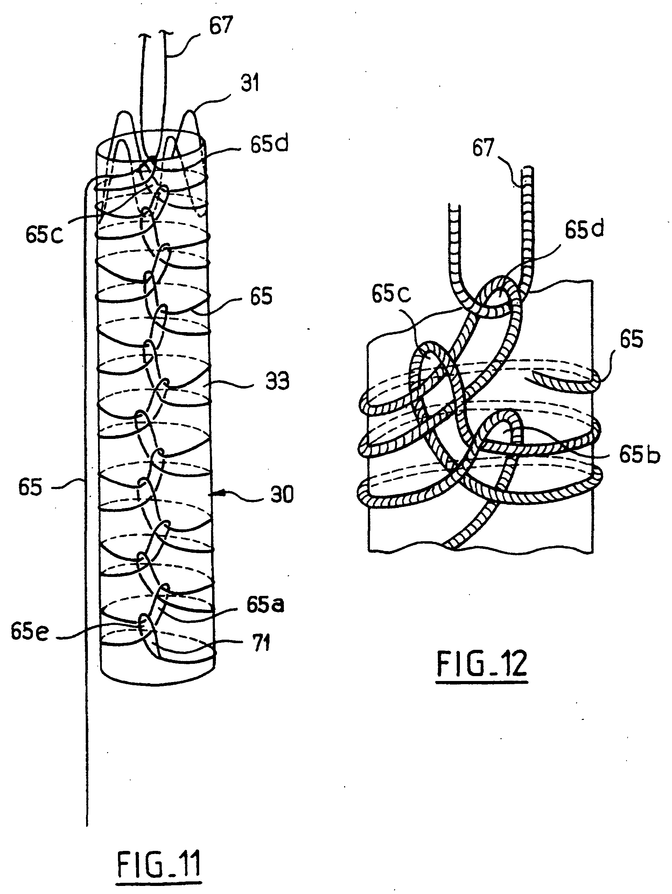

Dans la version des figures 9 à 13, le lien 65 a été enroulé de manière à définir une série de boucles dont trois ont été repérées en 65b, 65c, 65d, sur la figure 12. Ces boucles se tiennent toutes les unes les autres, jusqu'à l'avant-dernière (65c) et s'échelonnent le long du corps à maintenir pour former une chaîne.

Pour tenir le fil du côté de sa première boucle 65a, l'extrémité libre du fil la plus proche 65e a été préalablement nouée pour réserver un anneau 71 à l'intérieur duquel on a passé la première forme en boucle 65a.

Au-delà de la dernière boucle 65d, on voit toujours sur la figure 11 que le fil 65 repasse en arrière le long du corps puis, ensuite, le long des cathéters 52, 54 (figure 9) jusqu'à ressortir par le branchement latéral 64a sur la figure 8.

Quant au maintien de cette dernière boucle (distale) 65d, la figure 10 notamment montre qu'il peut être assuré par le passage du lien 67 à travers elle, les deux brins du fil 67 ainsi replié revenant ensuite en arrière pour passer entre les cathéters 56 et 58 jusqu'à ressortir par l'accès latéral 66a de la figure 8.

Dans l'exemple des figures 9 et 10, on a choisi de maintenir également le stent 31 serré extérieurement contre le cathéter 54.

Pour cela, le fil 69 a été d'abord plié sur lui-même en double, puis son extrémité courbée 69a glissée entre les cathéters 54 et 56, jusqu'à ressortir à l'extrémité distale 54a du cathéter 54 pour passer alors "dessus-dessous" dans les zigzags du stent 31, pour se terminer autour de la portion distale émergeante 56a du cathéter 56, en formant le noeud coulant 73, les deux brins réunis de fil 69 ressortant quant à eux par l'embranchement latéral 66b de la figure 8.

Une fois ces éléments ainsi mis en place, la gaine 52 va être glissée à l'intérieur de la gaine 49 (de diamètre approprié), de manière à reproduire la situation de la figure 7.

L'implant 30 est alors situé en regard de l'emplacement I repéré sur cette figure. Et l'embout 64 est alors en butée contre l'embout à valve 51.

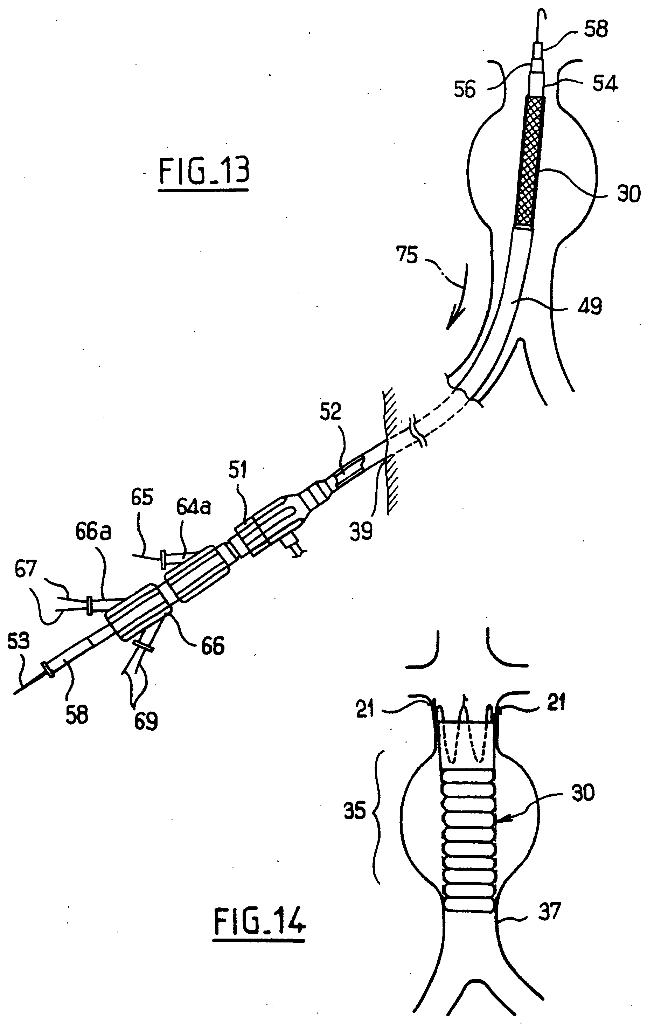

Tout en maintenant fixe en position le cathéter 58 et l'embout 66 porteur des cathéters 54, 56, le praticien tire alors vers l'arrière (dans le sens de la flèche 75 de la figure 13) l'ensemble formé par les gaines 49 et 52, de manière à se trouver dans la situation de la figure 13 qui laisse apparaître l'implant 30 toujours disposé avec ses liens et moyens de libération autour des cathéters plus centraux 54, 56 et 58.

En tirant sur l'un des deux brins du fil 67 sortant par 66a, ce fil peut être ensuite retiré, libérant ainsi de toute retenue la boucle distale 65d (voir figure 10) et par là même, la tenue des boucles en chaîne formée par le fil 65.

En tirant sur l'extrémité de ce fil qui ressort par 64a (figure 8), le praticien libère alors de sa retenue radiale au moins le corps de l'implant.

Si ce corps comprend la portion 33b déjà évoquée, celle-ci s'ouvre donc radialement pour reprendre sa forme tubulaire de la figure 5.

Dans l'hypothèse où le praticien craindrait que le retrait qu'il va maintenant effectuer du cathéter 56 entraîne, par l'intermédiaire du cathéter 54, un mouvement en particulier axial inopiné du stent 31 encore serré, on peut prévoir d'accorder un mouvement de translation axiale relatif entre les cathéters 54 et 56 (en scindant en outre, par exemple, en deux l'embout 66).

Quoi qu'il en soit, il faut que, par retrait vers l'arrière (flèche 75), le praticien obtienne un recul du cathéter 56 permettant à la partie du fil 69 l'entourant de s'échapper, libérant ainsi le noeud coulant 73. On peut alors par exemple tirer sur l'une des deux extrémités du fil 69 sortant par 66b pour retirer entièrement ce fil, libérant ainsi le stent 31 qui s'expanse alors radialement pour se plaquer contre la paroi en regard du vaisseau avec ses crochets qui s'ancrent dans cette paroi.

Il suffit alors au praticien de retirer entièrement les cathéters 54, 56 et 58, après quoi la voie d'abord 39 (et en particulier l'incision) peut être refermée, par exemple par sutures. L'implant 30 se trouve alors déployé dans le vaisseau, avec ses crochets 21 ancrés, comme sur la figure 14.

En alternative à la solution de nouage des figures 11 et 12, on peut retenir celle des figures 15 et 16.

Sur la figure 15, on note que si l'on trouve toujours un fil souple repéré 65' de maintien radial au moins du corps 33 de l'implant 30 à nouveau figuré schématiquement, on ne retrouve plus ni le fil de libération 67, ni le fil 69 de retenue radiale de l'armature de l'implant.

A la place, on remarque l'élément allongé 70 qui s'étend parallèlement à l'axe 30a de l'implant, à l'extérieur de son corps 33. En pratique, cet élément allongé 70 peut être une tige métallique ou plastique, de section de préférence aplatie, pour être peu encombrante radialement et éviter qu'elle forme un serpentin sous les efforts de cisaillement provoqués par le passage "alterné" du lien 65' autour de ladite barrette 70.

Bien qu'il soit délicat de décrire (en englobant les équivalents) la manière dont le lien 65' enlace le corps 33, tout en passant périodiquement autour de la barrette plate 70, on peut considérer qu'après avoir été noué, en partie intermédiaire, du côté de l'extrémité proximale 36 de l'implant (destinée à sortir la dernière de la gaine de pose 52 à l'intérieur du conduit d'implantation), tout en laissant libre l'un de ses brins (65'c), le lien 65' est enroulé en portion d'hélice autour du corps 33, avec un changement dans le sens d'enroulement de l'hélice à chaque fois qu'il passe autour de la barrette 70 (voir un changement de pente de cette hélice). On peut aussi considérer qu'ainsi le lien 65' définit, du fait des passages successifs avec changement d'orientation autour de la tige 70, une série d'épingles recourbées 65'a, 65'b, ... alternativement à gauche et à droite de la barrette et échelonnées axialement, jusqu'à ce qu'à son extrémité libre 65'd le lien forme un noeud non serré 66' autour de l'extrémité distale 70b de l'élément 70 qui s'étend donc sur la longueur voulue, depuis l'extérieur du corps du patient en direction de l'extrémité distale 38 de l'implant.

Pour libérer ce dernier, ou du moins son corps canalisant 33, l'implant alors serré par le lien 65' lui-même maintenu dans cette situation de retenue radiale par la tige 70, est placé dans la zone I de la figure 7. Une portion proximale de la tige 70 ressort suffisamment hors du corps du patient, à travers l'ensemble de pose déjà présenté, pour être manoeuvrable par le praticien.

Ce dernier, le moment venu, tire vers l'arrière la tige 70, dans le sens de la flèche 77 de la figure 15. Le noeud 66' s'échappe alors de l'extrémité distale de la tige 70, a priori en se dénouant, permettant ainsi à l'implant (ou du moins à son corps 33, ou équivalent) de pouvoir s'ouvrir naturellement (ou d'être ouvert par un stent), ceci sans effort créé sur lui et progressivement.

Soit en même temps qu'il retire entièrement la tige 70, soit via le brin 65'c qui ressort (par exemple, via la sortie latérale 64a de la figure 8), le praticien récupère tant le lien 65', alors défait, que la tige 70.

A noter que l'une et l'autre des versions des figures 11 et 15 permettent un dénouage progressif, uniquement dans un sens, du lien entourant le corps formant manchon de l'implant, ceci depuis l'extrémité distale de ce dernier qui sort en premier lors de la pose dans le conduit d'implantation, ce qui permet de bien contrôler l'ouverture radiale de l'implant, quand bien même la retenue radiale du ou des stents dont il peut être par ailleurs pourvu ne serait pas contrôlé par un lien mais uniquement par la gaine 52, tant que l'implant y est contenu.

On notera également que, tant le laçage des fils de retenue 65, 65' que leur "délaçage" s'opèrent ici dans un même sens, de préférence à une évolution dans deux sens opposés (qu'il s'agisse d'un rapprochement vers une zone centrale ou d'un écartement en direction des extrémités opposées de l'implant).

A noter encore que le système de lien(s) de l'invention pourrait même être utilisé sur des implants dépourvus d'armature de type "stent".

## Revendications

1. Système de réparation d'un conduit anatomique dans lequel circule un fluide, comprenant :
- un implant (1, 10, 20, 30) ayant un corps (3, 13, 23, 33) propre à canaliser le fluide selon un tube unique (3, 23, 33) ou ramifié (13) s'étendant globalement le long d'un axe principal de l'implant (1a, 10a, ...), au moins dans une configuration de ce dernier, lequel présente une première configuration pour être implanté dans le conduit, ou une seconde configuration, une fois implanté, la première configuration présentant un encombrement radial par rapport audit axe principal inférieur à la seconde configuration et ledit corps tubulaire ayant une longueur (L) suivant cet axe,
- un dispositif (40, 50) de pose de l'implant dans le conduit, le dispositif comprenant:
∗ au moins un lien de maintien (65, 65', 69) pour maintenir le corps de l'implant sensiblement dans sa première configuration,le(s) lien(s) définissant une succession de boucles se tenant les unes les autres (64a, 65b,65'a, 65'b) en s'échelonnant autour du corps, au moins sur une portion majeure de sa longueur (L), pour former une chaîne,
∗ et un moyen (67, 70) de libération du lien pour permettre à l'implant de passer de sa première configuration à sa seconde configuration,
**caractérisé en ce que** :
-- le système comprend en outre une gaine (52) pour la mise en place de l'implant dans le conduit, cette gaine présentant une extrémité distale à introduire le plus profondément dans le corps du patient à traiter, avec l'implant disposé dedans et maintenu dans sa première configuration,
-- le(s) lien(s) (65, 65') se termine(nt), vers l'extrémité distale de l'implant la plus proche de celle de la gaine, par un noeud (66') ou une boucle (65d) distal(e),
-- et le moyen de libération s'étend depuis l'extérieur du corps du patient jusque vers l'extrémité distale du corps de l'implant en bloquant là le noeud (66') ou la boucle (65d) distal(e) jusqu'à échappement dudit moyen de libération hors du noeud ou de la boucle, provoquant alors le passage à la seconde configuration, avec libération à partir de l'extrémité distale.

2. Système selon la revendication 1, **caractérisé en ce que** le moyen de libération comprend un élément allongé (70) flexible, ayant une rigidité supérieure à celle du(des) lien(s) et s'étendant, depuis l'extérieur de la gaine, au moins sur ladite portion majeure de la longueur (L) du corps, le lien passant sensiblement en épingle recourbée autour de l'élément allongé, périodiquement d'un côté et de l'autre de celui-ci, en s'échelonnant au moins sur ladite majeure portion de la longueur du corps jusqu'à former ledit noeud libérable (66') sensiblement à l'extrémité distale de liaison entre l'élément allongé et le lien considéré, permettant de libérer ce dernier par échappement du noeud vis-à-vis de l'élément allongé de libération (70).

## Claims

1. A system for repairing an anatomical duct in which a fluid circulates, comprising :
- an implant (1, 10, 20, 30) having a body (3, 13, 23, 33) adapted for channelling the fluid along one of a single tube (3, 23, 33) and a branched tube (13) extending generally along major axis (1a, 10a, ...)of the implant, at least in one configuration thereof, the implant having a first configuration in order to be implanted in the duct, or a second configuration, once implanted, the first configuration having a radial dimension overall with respect to the major axis which is smaller than the second configuration and the tubular body having a length (L) along the axis,
- a device (40, 50) for installing the implant in the duct, the device comprising :
* at least one retaining tie (65, 65', 69) to maintain the body of the implant substantially in its first configuration, the tie(s) defining a succession of loops holding on to one another (64a, 65b, 65'a, 65'b) while being spaced round the body, at least over a major portion of its length (L), to form a chain,
* and means (67, 70) for releasing the tie, to allow the implant to pass from its first configuration to its second configuration,
**characterized in that**
- the system according additionally comprises a sheath (52) for placing in position the implant in the duct, the sheath having a distal end to be introduced deepest into the body of the patient to be treated, with the implant placed inside, maintained in its first configuration.
- said at least one retaining tie ( 65, 65') terminates, towards the distal end of the implant closest to that of the sheath, in a distal knot (66') or loop (65d),
- and wherein the means for releasing extend from outside the body of the patient towards the distal end of the body of the implant, there locking the distal knot (66') or loop (65d) until an escape of said means for releasing from the knot or loop, then bringing about the passage to the second configuration, with release starting from the distal end.

2. The system according to Claim 1, **characterized in that** the means for releasing comprise a flexible elongate element (70) having a rigidity greater than that of said at least one retaining tie and extending, from outside the sheath, at least over said major portion of the length (L) of the body, said at least one tie passing substantially in a hairpin shape round the elongate element, periodically on one side and the other thereof, while being spaced at least over said major portion of the length of the body until it forms the releasable knot (66') substantially at the distal end of connection between the elongate element and said at least one tie, making it possible to release the tie by the escape of the knot from the elongate release element (70).

## Patentansprüche

1. System zum Ausbessern eines Körpergangs, in dem ein Fluid zirkuliert, das Folgendes umfasst:
- ein Implantat (1, 10, 20, 30) mit einem Körper (3, 13, 23, 33), der das Fluid entlang einer sich allgemein entlang einer Hauptachse des Implantats (1a, 10a, ...) erstreckenden einzigen Röhre (3, 23, 33) oder verzweigen Röhre (13) leitet, zumindest bei einer Konfiguration dieses Implantats, welches eine erste Konfiguration zum Implantieren in dem Gang oder eine zweite Konfiguration nach dem Implantieren aufweist, wobei die erste Konfiguration bezüglich der Hauptachse geringere Radialabmessungen aufweist als die zweite Konfiguration und der röhrenförmige Körper eine Länge (L) entlang dieser Achse aufweist,
- eine Vorrichtung (40, 50) zum Anbringen des Implantats in dem Gang, wobei die Vorrichtung Folgendes umfasst:
* mindestens ein Halteband (65, 65', 69) zum Halten des Körpers des Implantats im Wesentlichen in seiner ersten Konfiguration, wobei das (die) Band (Bänder) eine Folge von Schlaufen definiert (definieren), die, zumindest über einen Hauptteil der Länge (L) des Körpers verteilt, miteinander verbunden sind (64a, 65b, 65'a, 65'b), um eine Kette zu bilden,
* und ein Mittel (67, 70) zur Freigabe des Bands, um zu gestatten, dass das Implantat aus seiner ersten Konfiguration in seine zweite Konfiguration gelangt,
**dadurch gekennzeichnet, dass**:
- das System des Weiteren eine Hülse (52) zur Anordnung des Implantats in dem Gang enthält, wobei diese Hülse ein distales Ende, das tiefstmöglich in den Körper des zu behandelnden Patienten eingeführt werden soll, aufweist, wobei das Implantat darin angeordnet und in seiner ersten Konfiguration gehalten wird,
- das (die) Band (Bänder) (65, 65') am distalen Ende des Implantats, das dem der Hülse am Nächsten ist, durch einen distalen Knoten (66') oder eine distale Schlaufe (65d) abschließt (abschließen),
- und sich das Freigabemittel vom Äußeren des Körpers des Patienten bis zum distalen Ende des Körpers des Implantats erstreckt und dort den distalen Knoten (66') oder die distale Schlaufe (65d) bis zum Entfernen des Freigabemittels aus dem Knoten oder der Schlaufe verriegelt, wodurch dann der Übergang zur zweiten Konfiguration unter Freigabe vom distalen Ende aus bewirkt wird.

2. System nach Anspruch 1, **dadurch gekennzeichnet, dass** das Freigabemittel ein längliches, flexibles Element (70) umfasst, das eine größere Steifigkeit als die des (der) Bands (Bänder) aufweist und sich vom Äußeren der Hülse zumindest über den Hauptteil der Länge (L) des Körpers erstreckt, wobei sich das Band im Wesentlichen wie eine gekrümmte Haarnadel um das längliche Element herum, abwechselnd von einer Seite und von der anderen Seite davon erstreckt und sich dabei zumindest über den Hauptteil der Länge des Körpers verteilt, bis der freigebbare Knoten (66') im Wesentlichen am distalen Verbindungsende zwischen dem länglichen Element und dem betreffenden Band gebildet wird, wodurch Letzteres durch Entfernen des Knotens gegenüber dem länglichen Freigabeelement (70) freigegeben werden kann.
